# EUROPEAN PATENT SPECIFICATION

(11) **EP 2 556 804 B1**
(45) Date of publication and mention of the grant of the patent: **12.03.2014**
(21) Application number: 12180079.1
(22) Date of filing: 10.08.2012
(51) Int. Cl.: A61B 19/00

(54) **System for indicating positioning of an internal anatomical feature**
System zur Anzeige einer Position eines inneren anatomischen Merkmals
Système d'indication d'une position d'une caractéristique anatomique interne

(30) Priority: 12.08.2011 US 201161522794 P; 20.07.2012 US 201213553945
(43) Date of publication of application: 13.02.2013
(73) Proprietor: Covidien LP, Mansfield, MA 02048 (US)
(72) Inventor: Rockrohr, Brian, Waterbury, Connecticut 06705 (US)
(74) Representative: Maschio, Antonio

(56) References cited:
- WO-A1-2010/026357
- WO-A2-2007/094002
- US-A- 5 772 593
- US-A1- 2003 125 759
- US-A1- 2006 224 148
- US-A1- 2007 167 701
- US-A1- 2010 094 116

## Description

### Technical Field

The present disclosure relates to an apparatus for determining positioning of internal anatomical features. More particularly, the present disclosure relates to providing an internal probe to allow a surgeon to visually locate an internal anatomical feature to identify the location or edges of the internal anatomical feature on a display device.

### Background of Related Art

Image guided surgery has become more and more common, in part because of the ability of a surgeon to view internal images of a patient's anatomy and pre-plan a medical operation. In this way, for example, pre-acquired images of the anatomical body are used to plan the course of a medical procedure, whether the medical procedure is diagnostic, therapeutic or surgical in nature. The pre-acquired images may also be used, to some extent, during the medical procedure for orientation of the surgeon with respect to the internal anatomy of the patient.

The images of a patient's external or internal anatomy used in image guided surgery may be generated by, for example, computerized tomography (CT), magnetic resonance imaging (MRI), video, ultrasound and X-rays. Images may also be captured using angiography, single photon emission computer tomography and positron emission tomography (PET). In most cases, at least two, and generally more than two, images of the patient's internal anatomy are generated. The images are captured such that the relative position of the images is known. The images, along with information indicating the relative position of the images, may then be stored in a database including the pre-acquired images corresponding to the anatomical body of the patient at the time the images were captured.

Conventional imaging devices and methods distract the surgeon's focus and attention during the surgery, by directing the surgeon's focus and attention to the pre-acquired images, the pre-planned markings and the representations of the probes and instruments on the images. This may adversely affect the surgeon's hand-eye coordination and could result in the surgeon becoming disoriented. Additionally, it is difficult to accurately place an implant or a mesh evenly around the edges or perimeter of a defect (or portion of an internal anatomical feature). Surgeons have stressed the importance of detecting the margins around a defect for accurately placing the implant or mesh over the defect so that the implant or the mesh not only covers the defect, but also maintains an adequate margin of material around the all edges of the defect.

Accordingly, in order to use the pre-planned markings, the surgeon is required to constantly refer to the images and orient the images and pre-planned markings to the anatomical body during the course of the medical procedure. Thus, it is desirable to provide a system and method that determines the positioning of internal anatomical features without the sole reliance of a surgeon's attention to pre-acquired images.

WO 2007/094002 describes a system suitable for indicating positioning of an internal anatomical feature including a display device external to a subject and a marking device in the subject to identify points of interest, the display device, displaying the points of interest in response to a signal from the marking device.

### SUMMARY

The following presents a simplified summary of the claimed subject matter in order to provide a basic understanding of some aspects of the claimed subject matter. This summary is not an extensive overview of the claimed subject matter. It is intended to neither identify key or critical elements of the claimed subject matter nor delineate the scope of the claimed subject matter. Its sole purpose is to present some concepts of the claimed subject matter in a simplified form as a prelude to the more detailed description that is presented later.

Accordingly, a system for indicating positioning of an internal anatomical feature is provided in accordance with Claim 1. The system includes a display device external to a subject and a marking device positionable within the subject to identify one or more points of interest within a first region of interest. The display device is triggered to display the one or more points of interest in response to at least one signal received from the marking device.

The display device may be applied to an outer surface of the subject. In one exemplary embodiment, the display device is a physical sheet of reactive material draped over the subject. In yet another exemplary embodiment, the display device is a solution coated onto the subject. In yet another exemplary embodiment, the display device is a monitor.

In another exemplary embodiment, the marking device is an energy source inserted through an abdominal wall of the subject. In yet another exemplary embodiment, the marking device is at least one of an ultraviolet (UV) source, an infrared (IR) source, a laser source, and a visible light source inserted through an abdominal wall of the subject.

The display device is configured to collect and store the one or more points of interest identified by the marking device. Additionally, the display device may be manually marked by a user.

In another exemplary embodiment, the marking device and/or display device are reusable.

Accordingly, a system for indicating positioning of an internal anatomical feature is provided. The system includes a marking device positionable within a subject to identify one or more points of interest within a first region of interest, the marking device configured to collect and store coordinates of the one or more points of interest and a display device configured to display the one or more points of interest in response to at least one signal received from the marking device.

In one exemplary embodiment, the at least one signal collected and stored by the marking device corresponds to a number of continuous or non-continuous points used for generating a map of the first region of interest.

In another exemplary embodiment, the display device is a display unit including a monitor for displaying the map of the first region of interest. In yet another exemplary embodiment, the display device is a projector configured to project the map of the first region of interest onto a display surface or an outer surface of the subject.

Additionally, the marking device collects and stores one or more points corresponding to a perimeter of the implant, representing a second region of interest, which can be used to create an overlay map defining a position of the implant relative to the first region of interest.

An exemplary method not according to the invention is presented for indicating positioning of an internal anatomical feature, including placing a display device external to a subject, positioning a marking device within the subject to identify one or more points of interest within a first region of interest and triggering the display device to display the one or more points of interest in response to at least one signal received from the marking device.

Another exemplary method not according to the invention is presented for indicating positioning of an internal anatomical feature, including positioning a marking device within a subject to identify one or more points of interest within a first region of interest, permitting the marking device to collect and store coordinates of the one or more points of interest and displaying, via a display device, the one or more points of interest in response to at least one signal received from the marking device.

### BRIEF DESCRIPTION OF THE DRAWINGS

The accompanying drawings, which are incorporated in and constitute a part of this specification, illustrate examples of the disclosure and, together with a general description of the disclosure given above, and the detailed description of the examples given below, serve to explain the principles of the disclosure, wherein:
FIG. 1 is a system for indicating positioning of an internal anatomical structure, in accordance with the present disclosure;
FIG. 2 depicts a plurality of separate and distinct marking devices positionable within a subject to identify one or more points of interest, in accordance with the present disclosure;
FIG. 3 depicts a single marking device having a plurality of distal ends and being positionable within the subject to identify one or more points of interest, in accordance with the present disclosure;
FIGS. 4A-4E are top views of the system of FIG. 1, where the region of interest is displayed on the display device, in accordance with the present disclosure;
FIG. 5 is a system for indicating positioning of an internal anatomical structure, where the marking device is connected to a display unit for displaying a map of the region of interest, in accordance with the present disclosure;
FIG. 6 is a system for indicating positioning of an internal anatomical structure, where the marking device is connected to a projector configured to project a map of the region of interest, in accordance with the present disclosure; and
FIG. 7 illustrates the system of FIG. 1, where a mesh or implant is placed on the internal anatomical feature after the positioning of the internal anatomical feature has been determined, in accordance with the embodiments of the present disclosure.

The figures depict preferred embodiments of the present disclosure for purposes of illustration only. One skilled in the art will readily recognize from the following discussion that alternative embodiments of the structures and methods illustrated herein may be employed without departing from the principles of the present disclosure described herein.

### DETAILED DESCRIPTION

Examples of the presently disclosed apparatus will now be described in detail with reference to the drawings, in which like reference numerals designate identical or corresponding elements in each of the several views. As used herein, the term "distal" refers to that portion of the tool, or component thereof which is further from the user while the term "proximal" refers to that portion of the tool or component thereof which is closer to the user.

Reference will now be made in detail to examples of the present disclosure. While certain examples of the present disclosure will be described, it will be understood that it is not intended to limit the present disclosure. To the contrary, reference to examples of the present disclosure is intended to cover alternatives, modifications, and equivalents as may be included within the scope of the present disclosure as defined by the appended claims.

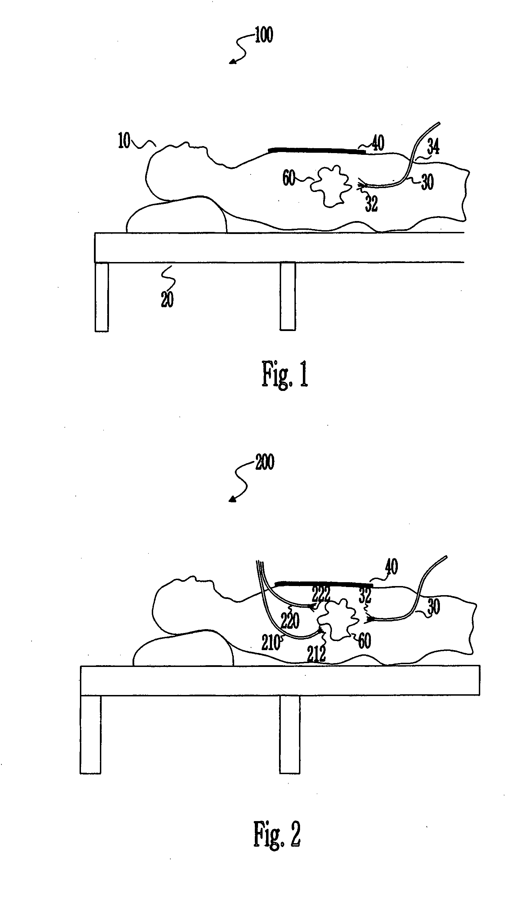

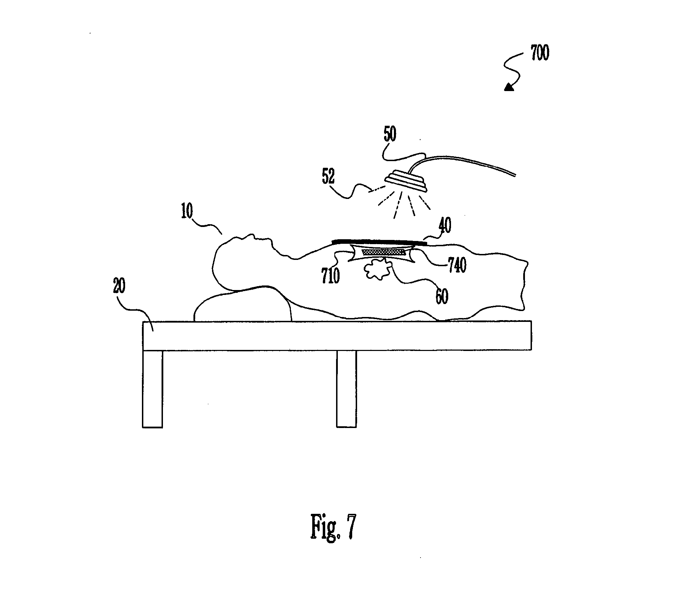

Referring to FIG. 1, there is disclosed a system 100 for use in minimally invasive surgery. The system 100 is configured to indicate positioning of an internal anatomical feature 60 (e.g., hernia) and for placing an implant or a mesh 710 (see FIG. 7) on the internal anatomical feature 60. In system 100, a subject 10 is placed on a surgical table 20. A surgeon may target the internal anatomical feature 60 of the subject 10 to be mapped. In order to map the internal anatomical feature 60, a display device 40 is placed external to the subject 10. The outer surface may be, for example, the abdomen of the subject 10. Then a marking device 30 is positioned within the subject 10 to identify one or more points of interest, within a region of interest. The distal end of the marking device 30 may include a signaling unit 32. Additionally, the marking device 30 may be inserted, for example, through an opening 34 of the abdomen of the subject 10. After the marking device 30 has been placed within the subject 10 and in close proximity to the region of interest of the anatomical feature 60 the display device 40 is signaled to display one or more points of interest in response to a signal from the marking device 30.

Therefore, the present disclosure relates to accuarately marking the placement of at least one internal anatomical feature 60 on the outside of the subject 10 to aid in the placement and attachment of a hernia mesh 710 (see FIG. 7). The system 100 includes two components, that is, a marking device 30 and a display device 40. The display device 40 may take the form of a physical sheet of material that is draped over the subject 10 or it may be a solution that is "painted" onto the patient's skin. One skilled in the art may contemplate any type of displaying unit or mechanism for displaying the internal anatomical feature 60.

The marking device 30 may be constructed, for example, as a laser pointer. However, the marking device 30 may be at least one of an ultraviolet (UV) source, an infrared (IR) source, a laser source, and a visible light source. The marking device 30 is used intra-abdominally to highlight the region of interest or the one or more points of interest.

Alternatively, the marking device 30 may be a physical probe that is placed adjacent the abdominal wall of the subject 10 at one or more points of interest. The display device 40 may either be activated automatically by the marking device 30 (e.g., by using a magnet in the marking device 30 to alter the appearance of the display device 40) or the surgeon may manually mark the external surface of the display device 40 to record each point of interest.

Alternatively, the marking device 30 may take the form of a bright light located in the abdominal space, effectively revealing the structure of interest by the shadows seen in the abdominal wall when observed externally from the subject 10. The marking device 30 may be used to highlight individual points or to draw continuous lines. In the embodiment, a second display device 740 is integrated into the hernia mesh 710 (see FIG. 7), representing another region of interest, thus allowing the surgeon to retrace the marks on the outside surface of the first region of interest back onto the mesh 710 itself. This effectively un-hides the relevant anatomical features 60 that are behind the mesh 710, as will be discussed below with reference to FIG. 7.

Moroever, the size, location, and orientation of the internal anatomical fetaure 60 may be determined by the system 100. By moving, displacing, manipulating or rotating the marking device 30, a plurality of data point relating to the internal anatomical feature 60 may be acquired in various planes. From the plurality of data points obtained, a reconstruction of locational information or a picture of the data or points or signals may be formed. One skilled in the art may contemplate a plurality of different methodologies for creating a map or determining the location or edges of the defect and the margin of material around the defect after placement of the mesh.

Therefore, in a first example, the present disclosure utilizes an internal probe (e.g., the marking device 30) to allow the surgeon to visually locate the defect and a methodology to allow the surgeon to identify the location or edges of the defect on a display device 40 positioned on the subject 10. In such an example, a beam of energy from the marking device may be transmitted through the abdomen (e.g., differentiating the defect by shining a brighter light in the visible spectrum through the defect) or it may be a different type of energy chosen to compliment the function of the display device 40. For example, a UV source may be used to fluoresce off a reactive material draped over the abdomen or off a solution "painted" on the patient's skin, as discussed above.

Referring to FIG. 2, a plurality of separate and distinct marking devices 30, 210, 220 positionable within the subject 10 to identify one or more points of interest, in accordance with the present disclosure is presented.

For sake of clarity, similar elements described with reference to FIG. 1 will not be described with reference to FIG. 2. The system 200 illustrates a plurality of marking devices 30, 210, 220 positioned within the subject 10 to identify the one or more points of interest within a region of interest. The distal end of the marking device 30 may include the signaling unit 32, as described in FIG. 1. However, in contrast to FIG. 1, additional marking devices 210, 220 may be placed within the subject 10 to identify the one or more points of interest. For example, a second marking device 210 including a signaling unit 212 and a third marking device 220 including a signaling unit 222 may be placed within the subject 10 through one or more incision points in the, for example, abdomen of the subject 10. The multiple marking devices 30, 210, 220 provide the surgeon with multiple angles of the internal anatomical feature 60 for aiding the surgeon in orienting or mapping the internal anatomical feature 60. As such, an accurate representation of the location or edges of the defect may be displayed on the display device 40 by manipulating a plurality of marking devices.

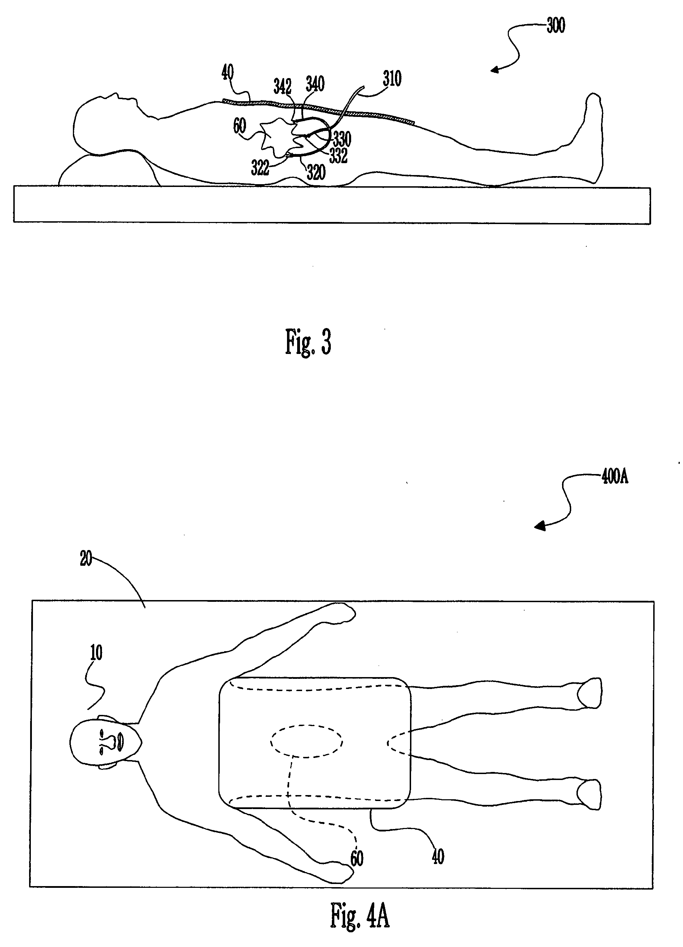

Referring to FIG. 3, a single marking device 310 having a plurality of distal ends 320, 330, 340 and positionable within the subject 10 to identify one or more points of interest, in accordance with the present disclosure is presented.

For sake of clarity, similar elements described with reference to FIG. 1 will not be described with reference to FIG. 3. The system 300 illustrates a single marking device 310 positioned within the subject 10 to identify one or more points of interest within a region of interest. The distal end of the marking device 310 may include three signaling units 320, 330, 340 each emitting, for example, a light beam 322, 332, 342, respectively. The surgeon may manipulate the distal ends of the single marking device 310 to pinpoint any desirable section or sections of the internal anatomical feature 60. Moreover, the signaling units 320, 330, 340 may be independently movable or actuable. As such, an accurate representation of the location or edges of the defect may be displayed on the display device 40 by manipulating a plurality of distal ends of a marking device. One skilled in the art may contemplate a plurality of different control mechanisms for controlling the distal ends of the marking device.

Referring to FIGS. 4A-4E, top views of the system of FIG. 1, where the region of interest is displayed on the display device 40 placed external to the subject 10, in accordance with the present disclosure are presented.

For sake of clarity, similar elements described with reference to FIG. 1 will not be described with reference to FIGS. 4A-4E. The system 400, as shown in FIG. 4A, illustrates a view of the internal anatomical feature 60 on the display device 40 positioned exterior to the subject 10. In operation, the marking device 30 (see FIG. 1) is inserted, for example, through the abdomen of the subject 10. After the marking device 30 has been placed within the subject 10 and in close proximity to the one or more points of interest, for example, the display device 40 is signaled or activated to display one or more points of interest in response to a signal from the marking device 30. Thus, the surgeon is in a position to view an outline or edges or outer periphery or outer perimeter of the internal anatomical feature 60 directly on the display unit 40. The outline of the internal anatomical feature 60 displayed on the display device 40 provides the surgeon with an accurate indication of where the mesh 710 (see FIG. 7) should be positioned on the internal anatomical feature 60.

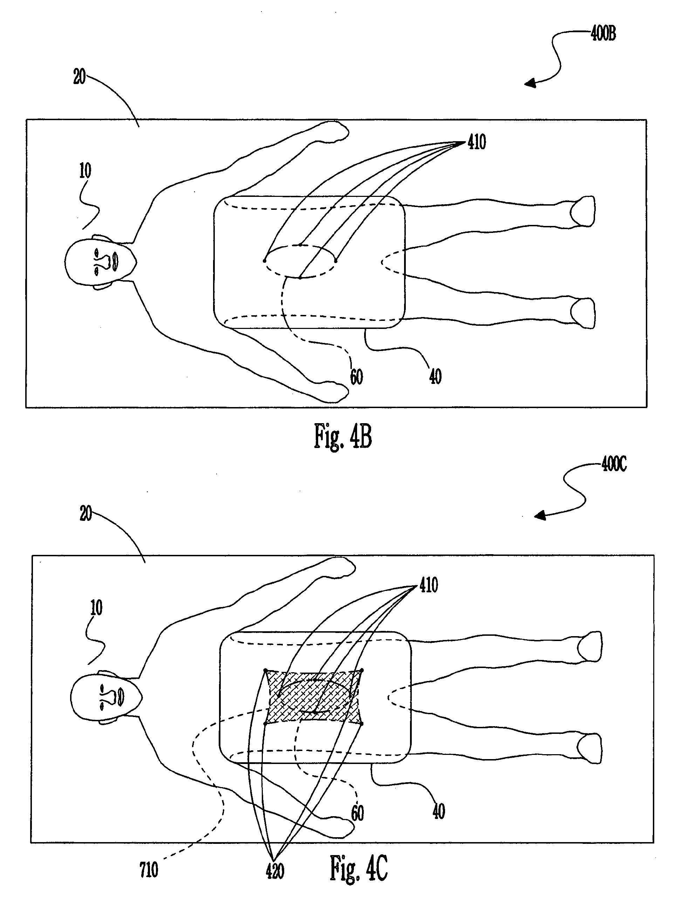

For example, referring to FIG. 4B points of interest 410 are displayed at opposed ends of the internal anatomical feature 60 in response to signals received from the marking device 30. In this instance, four points are displayed. FIG. 4C, further illustrates points of interest 420 related to the positioning of the mesh 710 and displayed in response to signals received from the marking device 30. In this instance, four points are displayed at each of the four edges of the mesh 710. One skilled in the art may contemplate a different number of points in different locations around the perimeter or edges of the internal anatomical feature 60 and/or mesh 710.

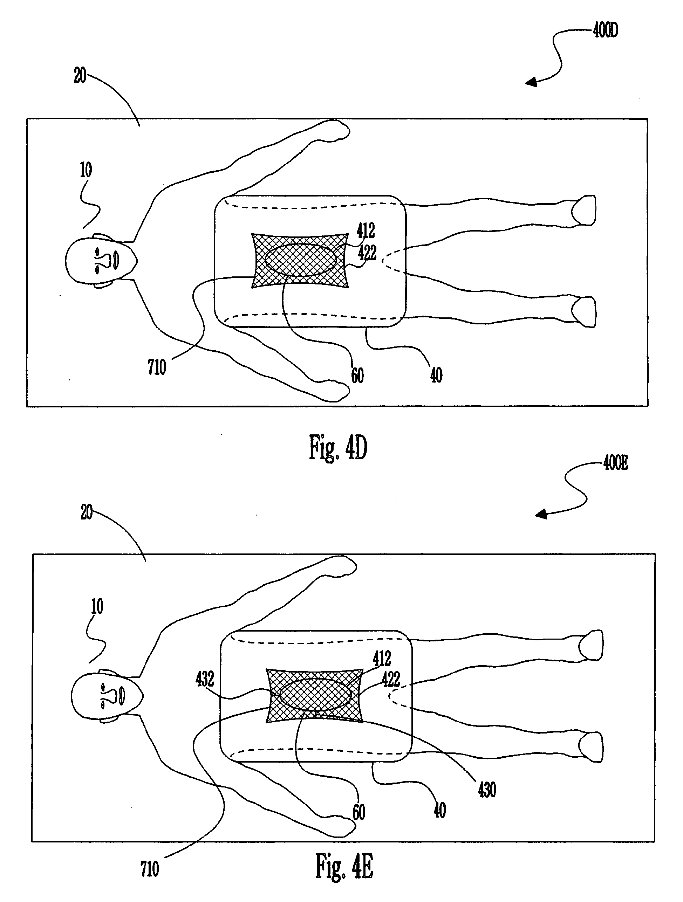

Thus, the edges of the internal anatomical feature 60 and/or mesh 710 are defined by points of interest in FIGS. 4B an 4C. However, the edges of the internal anatomical feature 60 and/or mesh 710 may also be defined by continuous lines. For example, FIG. 4D illustrates a continuous line 412 defining or identifying the perimeter or outline of the internal anatomical feature 60 and a continuous line 422 defining or identifying the perimeter or outline of the mesh 710. FIG. 4E also defines margins 430, 432. Margins 430, 432 indicate the length of material extending beyond the perimeter or edges of the internal anatomical feature 60. Of course, the margins 430, 432 may be varied in accordance with surgical applications performed.

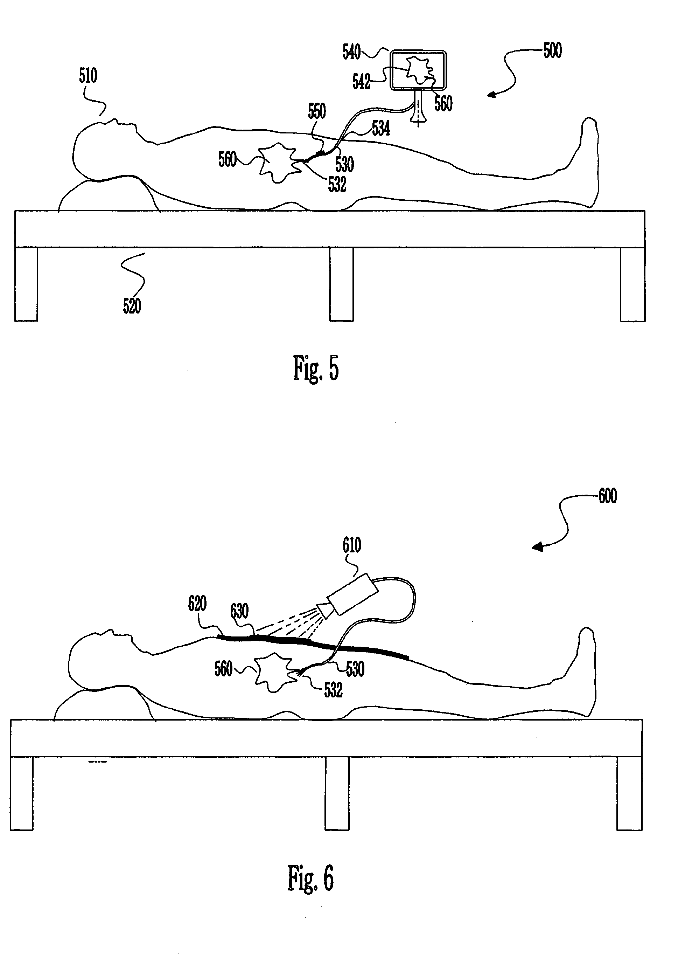

Referring to FIG. 5, a system 500 for indicating positioning of an internal anatomical structure 560, where the marking device 530 is connected to a display unit 540 for displaying a map of one or more regions of interest, in accordance with the present disclosure is presented.

In a second example, the system 500 is configured to indicate positioning of an internal anatomical feature 560 and for placing a mesh 710 (see FIG. 7) on the internal anatomical feature 560. In system 500, a subject 510 is placed on a surgical table 520. A surgeon may target the internal anatomical feature 560 of the subject 510 to be mapped. In order to map the internal anatomical feature 560, a display unit 540 is placed within close proximity to the subject 510. Then a marking device 530 is inserted into the subject 510 to identify one or more points of interest of a region of interest of the internal anatomical feature 560. The distal end of the marking device 530 may include a signaling unit 532. Additionally, the marking device 530 may be inserted, for example, through an opening 534 of the abdomen of the subject 510. The marking device 530 is placed within the subject 510 and in close proximity to the region of interest and/or one or more points of interest of the anatomical feature 560. The proximal end of the marking device 530 is connected to a display unit 540. The display unit 540 includes a screen 542 for displaying one or more points of interest related to the internal anatomical feature 560. The display unit 540 may be connected to the marking device 530 via wired or wireless means. One skilled in the art may contemplate a plurality of different wired or wireless connecting mechanisms configured to enable electrical and mechanism communication between all the components of the system 500.

Additionally, the marking device 530 may also be equipped with a recording unit 550 for recording one or more data points related to the one or more points of interest of the anatomical feature 560. The recording unit 550 may store the recording locally in a memory unit (not shown) or may transmit the recordings to external memory units. One skilled in the art may contemplate a plurality of different storage configurations for collecting and storing data related to the internal anatomical feature 560.

Therefore, the probe or marking device 530 may register its coordinates, via the recording unit 550, a number of points or continuous data or signals that may be used to create a map or model of the abdominal wall and/or the defect. Furthermore, the display unit 540 may be a monitor connected to a computer or software that processes and/or analyzes the data collected.

Additionally, either system 100 (see FIG. 1) or 500 (see FIG. 5) may add a second step or round of measurements to confirm that the placement of the mesh or patch or implant has adequately covered the defect and to ensure that the surgeon has adequate margins of material around all edges of the defect. For example, as discussed above with reference to FIGS. 4B-4E, in order to determine accurate placement, margins may be identified (see FIG. 4E) when either points of interest (see FIGS. 4B and 4C) or continuous lines (see FIGS. 4D and 4E) are used to define edges or the perimeter of the internal anatomical feature 60 and the mesh 710.

In the first example, a beam may be used to mark the location of the mesh or implant onto the display device, thereby allowing the surgeon to compare the location of the mesh or implant after the initial placement (e.g., after the stay sutures are placed), but before final fixation with tacks, sutures or other methods known in the art, with the location of the defect itself, as previously marked or displayed onto the display device.

For example, in the preferred embodiment of the invention, the internal probe may record the perimeter or edges or outline of the mesh or patch or implant to create a second "over-lay" map or model showing the position of the mesh relative to the defect. The preferred embodiment will be described in more detail with reference to FIG. 7.

Referring to FIG. 6, a system 600 for indicating positioning of an internal anatomical structure 560, where the marking device 530 is connected to a projector 610 configured to project a map 630 of the region of interest and/or one or more points of interest, in accordance with the present disclosure is presented.

For sake of clarity, similar elements described with reference to FIG. 5 will not be described with reference to FIG. 6. In this example, the system 600 is configured to indicate positioning of an internal anatomical feature 560 and for placing a mesh 710 (see FIG. 7) on the internal anatomical feature 560. In system 600, a subject 510 is placed on a surgical table 520. A surgeon may target the internal anatomical feature 560 of the subject 510 to be mapped. In order to map the internal anatomical feature 560, a display device 620 is dimensioned and arranged to be placed on an outer surface of the subject 510. A marking device 530 is then positioned within the subject 510 to identify one or more points of interest of the internal anatomical feature 560. The distal end of the marking device 530 may include the signaling unit 532. The marking device 530 is placed within the subject 510 and in close proximity to the region of interest and/or one or more points of interest of the anatomical feature 560. The proximal end of the marking device 530 is connected to a projector 610 that projects the map or model back onto the surface 630 of the patient's skin. One skilled in the art may contemplate projecting an outline of the internal anatomical feature 560 not only on the subject 510, but also on a wall or screen. The projector 610 may transmit, with wires or wirelessly, the data points collected to any external units for storage and further processing.

It is noted that processing the data points to generate a map or image may be performed via one or more processors running software on, for example, a general purpose computer, special purpose computer, or special purpose processer. Various general-purpose systems may also be used with programs in accordance with the teachings herein, or it may prove convenient to construct more specialized apparatus to perform the required method steps. The required structure for a variety of these systems will appear from the description above.

For example, examples as disclosed herein may also include computer-readable media for carrying or having computer-executable instructions or data structures stored thereon.

Referring to FIG. 7, a mesh 710 is placed on the internal anatomical feature 760 after the positioning of the internal anatomical feature 760 has been determined, in accordance with the embodiments of the present disclosure.

For sake of clarity, similar elements described with reference to FIG. 1 will not be described with reference to FIG. 7. In system 700, the mesh 710 is shown positioned on the internal anatomical feature 760.

In the embodiment of the present invention, the one or more points of interest of the first region of interest are projected by an energy source 50 back onto a secondary display device 740 incorporated within or embedded within or integrated with the implant or mesh 710. In other words, the first region of interest may be mapped back onto the mesh or second region of interest to aid the surgeon in visually determining from inside the patient accurate placement of the implant or mesh so as to ensure adequate margins of material around all edges of the defect. Thus, feedback received from inside the body of the subject may be displayed back onto the outside of the body of the subject, and vice versa. As a result, the secondary energy source 50 allows the remapping of certain points of interest back onto the mesh 710 in order to "unhide" the internal anatomical feature 60 and provide the surgeon (e.g., through an endoscope inside the abdomen) with the opportunity to "see" or "view" the internal anatomical feature 60 through the mesh 710.

Stated otherwise, a first map of the internal anatomical feature (or one or more points of interest) may be obtained via the marking device and external display device. Then, a second map of the internal anatomical feature (or one or more points of interest) may be obtained from the implant or mesh itself, which has a display device integrated thereon. The first map and the second map may be overlaid on each other to aid the surgeon in accurately placing the implant or mesh onto the defect. The first map may be overlaid on the second map or the second map may be overlaid on the first map based on the surgical procedure and/or surgeon's discretion. Therefore, once the implant or mesh has been placed on the defect, the integrated display device may provide feedback information to the surgeon so that the surgeon may be provided with a second chance to adjust the implant or mesh (after it has been placed) based on a comparison between the second map and the first map.

In summary, the systems and methods of the exemplary embodiments of the present disclosure are suitable for use in surgery for locating specific sub-surface regions of the anatomy and so that the specific region may be identified and targeted for surgical operations. Stated otherwise, the systems and methods of the present disclosure provide for accurate location of a position on the patient's surface anatomy that corresponds to an imaged subsurface or deep structure. A deep structure is, for example, a portion of the internal anatomy of a patient which is identifiable from surrounding structures by its differential radiotranslucency and which is medically of interest.

One advantage of the exemplary embodiments of the present disclosure is that the images projected by the image projecting device may be marked onto the patient. This may be performed by having an image projecting device, which emits radiation that may mark a patient. In a similar manner, use of an ultraviolet (UV) laser may be used to leave a mark on the skin of the patient corresponding to markings. Likewise, use of a photo-reactive ink, which perceptively changes in response to radiation emitted by the image projecting device may be used to temporarily mark the patient with markings. The photo-reactive ink may be either thermochromic ink, which reacts to infrared radiation, or, photo-chromic ink which may react to different types of radiation, such as ultraviolet radiation. The photo-reactive ink may be embedded in a material, such as plastic, which is draped over the patient and then marked with the radiation emitted by the image projecting device, rather than applying the substance to the surface of the patient. In this way, the photo-reactive ink embedded in the marking material would perceptively change and the image would appear on the marking material, rather than the image appearing on a substance applied to the patient.

In an alternative embodiment, the image or images are projected substantially normal to a surface of the corresponding locations or a portion of the corresponding locations. In this way, by projecting the image substantially normal to a surface of the corresponding locations of the anatomical body, a clearer image is produced and optical distortions are reduced.

It is also understood that while the present disclosure has been described in some embodiments as comprising a coherent beam of light, the present disclosure is not limited to coherent beams of light, but rather could comprise other beams of light, such as collimated beams of light.

Additionally, it is contemplated that the marking devices of the exemplary embodiments of the present disclosure are reusable. In an alternative embodiment, the internal probes of the exemplary embodiments of the present disclosure may also include a disposable covering.

It understood that while the present disclosure has been described in terms of the anatomical body of the human, the systems and methods are not limited to use on humans. Rather, the systems and methods of may be used in veterinary and other applications where internal anatomical features are displayed or projected onto the corresponding locations of the objects from which the images were acquired.

The word "exemplary" is used herein to mean "serving as an example, instance, or illustration." Any embodiment described herein as "exemplary" is not necessarily to be construed as preferred or advantageous over other embodiments.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of presently disclosed embodiments. Thus the scope of the embodiments should be determined by the appended claims and their legal equivalents, rather than by the examples given.

Persons skilled in the art will understand that the devices and methods specifically described herein and illustrated in the accompanying drawings are non-limiting exemplary embodiments. The features illustrated or described in connection with one exemplary embodiment may be combined with the features of other embodiments. Such modifications and variations are intended to be included within the scope of the present disclosure. As well, one skilled in the art will appreciate further features and advantages of the present disclosure based on the above-described embodiments. Accordingly, the present disclosure is not to be limited by what has been particularly shown and described, except as indicated by the appended claims.

## Claims

1. A system (100, 200, 300, 400, 500, 600, 700) for indicating positioning of an internal anatomical feature (60, 560, 760), the system comprising:
a display device (40, 540, 610, 620) external to a subject (10, 510); and
a marking device (30, 210, 220, 310, 530) positionable within the subject to identify one or more points of interest within a first region of interest;
wherein the display device displays the one or more points of interest (410) in response to at least one signal received from the marking device,
**characterised by** a second display device (740), integrated into an implant (710), so as to allow a user to retrace marks from the first region of interest back onto the implant.

2. The system according to Claim 1, wherein the display device is applied to an outer surface of the subject.

3. The system according to Claim 2, wherein the display device is a physical sheet of reactive material draped over the subject.

4. The system according to Claim 2, wherein the display device is a solution coated onto the subject.

5. The system according to Claim 2, wherein the display device is a monitor.

6. The system according to any preceding claim, wherein the marking device is an energy source inserted through an abdominal wall of the subject.

7. The system according to any preceding claim, wherein the marking device includes at least one of an ultraviolet (UV) source, an infrared (IR) source, a laser source, and a visible light source inserted through an abdominal wall of the subject.

8. The system according to any preceding claim, wherein the display device is configured to collect the one or more points of interest identified by the marking device.

9. The system according to any preceding claim, wherein the one or more points of interest are signaled manually by a user.

10. The system according to any preceding claim, wherein the marking device and/or display device are reusable.

11. A system according to any preceding claim, wherein the marking device is configured to record coordinates of the one or more points of interest..

12. The system according to Claim 11, wherein the at least one signal received from the marking device corresponds to a number of continuous or non-continuous points used for generating a map of the first region of interest.

13. The system according to Claim 11or Claim 12, wherein the display device is a display unit including a monitor for displaying the map of the first region of interest.

14. The system according to Claim 11 or Claim 12, wherein the display device is a projector configured to project the map of the first region of interest onto an outer surface of the subject.

15. The system according to any one of claims 11 to 14, wherein the marking device records one or more points corresponding to a perimeter of an implant, representing a second region of interest, defining a position of the implant relative to the first region of interest.

## Patentansprüche

1. System (100, 200, 300, 400, 500, 600, 700) zum Anzeigen der Positionierung eines inneren anatomischen Merkmals (60, 560, 760), wobei das System Folgendes umfasst:
eine Anzeigeeinrichtung (40, 540, 610, 620) außerhalb eines Subjekts (10, 510) und
eine Markierungseinrichtung (30, 210, 220, 310, 530), die innerhalb des Subjekts angeordnet werden kann, um einen oder mehrere Punkte von Interesse innerhalb eines ersten Bereichs von Interesse zu kennzeichnen,
wobei die Anzeigeeinrichtung den einen oder die mehreren Punkte von Interesse (410) als Reaktion auf wenigstens ein von der Markierungseinrichtung empfangenes Signal anzeigt,
**gekennzeichnet durch** eine zweite Anzeigevorrichtung (740), die in ein Implantat (710) integriert ist, um so einem Anwender zu ermöglichen, Markierungen aus dem ersten Bereich von Interesse auf dem Implantat wieder aufzufinden.

2. System nach Anspruch 1, wobei die Anzeigeeinrichtung auf eine Außenfläche des Subjekts aufgebracht wird.

3. System nach Anspruch 2, wobei die Anzeigeeinrichtung eine physische Bahn eines reaktiven Materials, die über das Subjekt gedeckt wird, ist.

4. System nach Anspruch 2, wobei die Anzeigeeinrichtung eine auf das Subjekt aufgetragene Lösung ist.

5. System nach Anspruch 2, wobei die Anzeigeeinrichtung ein Monitor ist.

6. System nach einem der vorhergehenden Ansprüche, wobei die Markierungseinrichtung eine durch eine Bauchwand des Subjekts eingeführte Energiequelle ist.

7. System nach einem der vorhergehenden Ansprüche, wobei die Markierungseinrichtung wenigstens eines von einer Ultraviolett- (UV-) Quelle, einer Infrarot- (IR-) Quelle, einer Laserquelle und einer Quelle sichtbaren Lichts, eingeführt durch eine Bauchwand des Subjekts, einschließt.

8. System nach einem der vorhergehenden Ansprüche, wobei die Anzeigeeinrichtung dafür konfiguriert ist, den einen oder die mehreren durch die Markierungseinrichtung gekennzeichneten Punkte von Interesse zu sammeln.

9. System nach einem der vorhergehenden Ansprüche, wobei der eine oder die mehreren Punkte von Interesse von Hand durch einen Anwender mitgeteilt werden.

10. System nach einem der vorhergehenden Ansprüche, wobei die Markierungseinrichtung und/oder die Anzeigeeinrichtung wiederverwendbar sind.

11. System nach einem der vorhergehenden Ansprüche, wobei die Markierungseinrichtung dafür konfiguriert ist, Koordinaten des einen oder der mehreren Punkte von Interesse aufzuzeichnen.

12. System nach Anspruch 11, wobei das wenigstens eine von der Markierungseinrichtung empfangene Signal einer Anzahl von zusammenhängenden oder nicht zusammenhängenden Punkten, die zum Erzeugen einer Karte des ersten Bereichs von Interesse verwendet werden, entspricht.

13. System nach Anspruch 11 oder Anspruch 12, wobei die Anzeigeeinrichtung eine Anzeigeeinheit, die einen Monitor zum Anzeigen der Karte des ersten Bereichs von Interesse einschließt, ist.

14. System nach Anspruch 11 oder Anspruch 12, wobei die Anzeigeeinrichtung ein Projektor, der dafür konfiguriert ist, die Karte des ersten Bereichs von Interesse auf eine Außenfläche des Subjekts zu projizieren, ist.

15. System nach einem der Ansprüche 11 bis 14, wobei die Markierungseinrichtung einen oder mehrere Punkte aufzeichnet, die einer Umgrenzung eines Implantats entsprechen, das einen zweiten Bereich von Interesse darstellt, der eine Position des Implantats im Verhältnis zu dem ersten Bereich von Interesse definiert.

## Revendications

1. Système (100, 200, 300, 400, 500, 600, 700) pour indiquer la position d'une caractéristique anatomique interne (60, 560, 760), le système comprenant :
un dispositif d'affichage (40, 540, 610, 620) à l'extérieur d'un sujet (10, 510) ; et
un dispositif de marquage (30, 210, 220, 310, 530) positionnable à l'intérieur du sujet pour identifier un ou plusieurs points d'intérêt à l'intérieur d'une première région d'intérêt ;
dans lequel le dispositif d'affichage affiche le ou les points d'intérêt (410) en réponse à au moins un signal reçu du dispositif de marquage,
**caractérisé par** un second dispositif d'affichage (740), intégré dans un implant (710), afin de permettre à un utilisateur de retracer des marques issues de la première région d'intérêt sur l'implant.

2. Système selon la revendication 1, dans lequel le dispositif d'affichage est appliqué à une surface externe du sujet.

3. Système selon la revendication 2, dans lequel le dispositif d'affichage est une feuille physique de matériau réactif drapée sur le sujet.

4. Système selon la revendication 2, dans lequel le dispositif d'affichage est une solution enduite sur le sujet.

5. Système selon la revendication 2, dans lequel le dispositif d'affichage est un moniteur.

6. Système selon l'une quelconque des revendications précédentes, dans lequel le dispositif de marquage est une source d'énergie insérée à travers une paroi abdominale du sujet.

7. Système selon l'une quelconque des revendications précédentes, dans lequel le dispositif de marquage comprend au moins une d'une source de rayonnement ultraviolet (UV), d'une source de rayonnement infrarouge (IR), d'une source laser et d'une source de lumière visible insérée à travers une paroi abdominale du sujet.

8. Système selon l'une quelconque des revendications précédentes, dans lequel le dispositif d'affichage est configuré pour collecter le ou les points d'intérêt identifiés par le dispositif de marquage.

9. Système selon l'une quelconque des revendications précédentes, dans lequel le ou les points d'intérêt sont signalés manuellement par un utilisateur.

10. Système selon l'une quelconque des revendications précédentes, dans lequel le dispositif de marquage et/ou le dispositif d'affichage sont réutilisables.

11. Système selon l'une quelconque des revendications précédentes, dans lequel le dispositif de marquage est configuré pour enregistrer les coordonnées du ou des points d'intérêt.

12. Système selon la revendication 11, dans lequel l'au moins un signal reçu du dispositif de marquage correspond à un nombre de points continus ou non continus utilisés pour générer une carte de la première région d'intérêt.

13. Système selon la revendication 11 ou la revendication 12, dans lequel le dispositif d'affichage est une unité d'affichage comportant un moniteur pour afficher la carte de la première région d'intérêt.

14. Système selon la revendication 11 ou la revendication 12, dans lequel le dispositif d'affichage est un projecteur configuré pour projeter la carte de la première région d'intérêt sur une surface externe du sujet.

15. Système selon l'une quelconque des revendications 11 à 14, dans lequel le dispositif de marquage enregistre un ou plusieurs points correspondant à un périmètre d'un implant, représentant une seconde région d'intérêt, définissant une position de l'implant par rapport à la première région d'intérêt.
